# EUROPEAN PATENT APPLICATION

(11) **EP 1 598 667 A1**
(43) Date of publication of application: **23.11.2005**
(21) Application number: 04708512.1
(22) Date of filing: 05.02.2004
(51) Int. Cl.: G01N 31/00, G01N 31/22

(54) **QUALITY CONTROL METHOD FOR ARTICLE AND OXYGEN DETECTING AGENT FOR USE THEREIN**

(30) Priority: 07.02.2003 JP 2003031409; 21.11.2003 JP 2003392517
(71) Applicant: Powdertech Co., Ltd., Kashiwa-shi, Chiba 277-0872 (JP)
(72) Inventor: KODAMA, Ryuichi, Kashiwa-shi, Chiba 2770872 (JP); TAMURA, Minoru, Kashiwa-shi, Chiba 2770872 (JP); SHINOHARA, Kazuhiko, Kashiwa-shi, Chiba 2770872 (JP); ENDO, Hiroshi, Kashiwa-shi, Chiba 2770872 (JP); WADA, Toshitaka, Kashiwa-shi, Chiba 2770872 (JP); TANAKA, Shigeru, Kashiwa-shi, Chiba 2770872 (JP)
(74) Representative: Golding, Louise Ann
(86) International application number: PCT/JP2004/001216
(87) International publication number: WO 2004/077049

(57) **Abstract**

There is provided a method for controlling the quality of a product, **characterized in that** a product and an oxygen detecting agent having a predetermined sensitivity corresponding to the kind of the product and quality control conditions for the product are provided in combination and the quality of the product is controlled by taking advantage of such a phenomenon that, when a predetermined period of time corresponding to the kind of the product and the quality control conditions for the product has elapsed, the oxygen detecting agent undergoes a change in color as a result of the detection of the concentration of oxygen, which has infiltrated the oxygen detecting agent, by the oxygen detecting agent. There is also provided an oxygen detecting agent **characterized by** comprising an oxygen detecting agent body and a film that hermetically covers said body and has a predetermined oxygen permeability and/or carbon dioxide permeability corresponding to the kind of a product, of which the quality is to be controlled, and the quality control conditions for the product.

## Description

### TECHNICAL FIELD

The present invention relates to a method for controlling the quality of a product, which is suitable for use in a method for controlling the freshness of food such as a gas replaced packaged food, and an oxygen detecting agent for use in said method.

More particularly, the present invention relates to a method for controlling the quality of a product typified by a method for controlling the freshness of food, and an oxygen detecting agent for use in said method, in which, when a detecting agent is applied directly or indirectly to a product typified by food, for example, consumers or food transactors can very easily determine the freshness of the food as a measure of merchandise choice and discrimination or the like, by taking advantage of such a phenomenon that, after display of food or the like on a display shelf in a supermarket or the like, when a predetermined quality preservation period corresponding to the kind of the food or the like has elapsed, the detecting agent undergoes a change in color.

### BACKGROUND ART

Oxygen detecting agents have hitherto been extensively used, for example, in the field of food, the field of printing ink, the field of electric appliances and machinery and appliances, and the field of pharmaceutical preparations and medical supplies, for example, for the determination of quality maintenance and quality control of various products within packaging containers, for example, food products, inks, electric appliances and machinery and appliances, and pharmaceutical preparations and medical supplies.

For example, in the field of food products, the oxygen detecting agent is mainly used for food products to be stored for a long period of time. Specifically, for example, in order to prevent an oxidative deterioration of fats and oils and vitamins contained in food, to prevent damage by vermin, to prevent a change in color and gloss, and to prevent growth of fungi and bacteria, the oxygen detecting agent is always enclosed together with a deoxidizer (an oxygen absorbing agent) in a gas impermeable hermetically sealable packaging bag containing food therein. In this case, the inside of the packaging bag is maintained in a substantially oxygen-free state for a long period of time, and, independently of the kind of the food and the like, the oxygen detecting agent detects a predetermined amount of oxygen which enters the interior of the packaging bag from the ambient environment and uniformly undergoes a change in color, and any oxygen detecting agent which undergoes a change in color under different conditions, for example, depending upon the kind of food have not been proposed in the art.

Specifically, in the prior art technique, food, a deoxidizer, and an oxygen detecting agent are enclosed in a packaging bag. With the elapse of a long-term preservation period of the food, oxygen enters, from the external environment, the interior of the packaging bag, for example, through a hermetically sealed part and pinholes formed in the packaging bag. This oxygen is adsorbed on or reacted with the deoxidizer to reduce or remove oxygen within the packaging bag. For example, upon a deterioration in performance of the deoxidizer, the concentration of oxygen within the packaging bag is increased. The amount of oxygen present within the packaging bag (that is, oxygen which has not been adsorbed on or reacted with the deoxidizer) is confirmed based on whether or not the color of the oxygen detecting agent has changed. This is used as a measure of visual determination of the freshness of the food contained in the packaging bag (see, for example, Japanese Patent Laid-Open Nos. 308342/2002, 342235/2000, 039429/2000, 276888/1999, and 190729/1999, and Japanese Utility Model Laid-Open No. 85623/1993).

However, it should be noted that various food products are on the market, and, in addition, the average number of days required from distribution routes and time point of production of individual food products to time point of eating of the food by consumers are also various, and the temperature (product temperature) of the food during storage and transport or during display, and the form of display of the food in retailing stage in supermarkets or the like are also various. Therefore, the deoxidizer or the oxygen detecting agent is not always enclosed in all the food products.

For example, ready-to-eat daily dishes are in many cases placed on a food display shelf in a supermarket or the like in a short period, that is, for about 2 to 3 days at room temperature (15 to 25°C) after the preparation thereof, and are purchased and consumed by consumers in the two or three day period. In the case of such food products that are sold on the premise that they are consumed in a short period after the preparation thereof, neither the deoxidizer nor the oxygen detecting agent is enclosed.

Further, regarding raw animal meat, if necessary, the deoxidizer and the oxygen detecting agent are enclosed, and, for example, long-term storage in a frozen state at such a temperature that does not substantially cause a deterioration in quality (for example: about -40°C to -18°C), or chilled transport at that temperature is conducted. Thereafter, the raw animal meat is divided into small parts (a few hundred grams to a few kilograms) which are then individually packed (or otherwise, in a breaking-down packaging process, the raw animal meat is previously divided into small parts which are then individually packed). These small divided packs are in many cases placed on a food display shelf (cabinet) in a supermarket or the like for about 3 to 4 days at a temperature of about 5 to 6°C, and; in a few days during which good freshness of the raw meat can be maintained, are purchased and consumed by consumers. In the case of the raw animal meat, in the stage of display on a food display shelf in a supermarket, neither the deoxidizer nor the oxygen detecting agent is enclosed, probably because the raw animal meat is sold on the premise that the raw animal meat is consumed in a short period.

When consumers buy products such as food placed on the display shelf or the like,' most consumers choose the food product based on the price or quality of the food. In this case; consumers grasp the quality of the food based on the appearance of the food and, in addition, notations affixed to each product, such as date of packing and the date of quality preservation period and use these data as criteria for the choice of the food product.

Further, when consumer buys food products which are then stored in a home refrigerator or the like and are eaten before the use-by date of the food product, the consumer consumes the food products within the refrigerator in the order of the nearest use-by date of the food product based on the appearance and date of packing of the food product and the like.

Further, as with consumers, for example, persons responsible for controlling merchandise or employees in retail stores collect individual data, for example, the appearance of food products and, in addition, notations affixed to each food product such as the date of packing and the date of quality preservation period and use these data as criteria for exchange of the food product and disposal or the like.

In such food control, for consumers and employee of retail stores or the like, in confirming the quality of each food product, holding the food product in his or her hand for reading, for example, stamped small letters or numerals indicating the date of packing and the like is troublesome. In this case, if, for example, whether or not the food product is still within the quality preservation period can be visually determined in a simpler manner, for example, based on a change in color of a label, then this can save labor for the choice of food product and is convenient. In particular, that whether or not the food is within the quality preservation period can be visually determined in a simple manner, for example, based on a change in color of a label, is convenient for consumers, at the time of purchase of the food product, as well as after the purchase of the food product. Specifically, after the purchase of the food product, the consumer can store the food product in his or her home refrigerator or the like and can make an optimal bill of fare so that the food product which is near the use-by date can be fully consumed.

In the prior art, in the case of food products which are provided on the premise that they are stored and controlled for a long period of time, for example, confectionery, a deoxidizer and an oxygen detecting agent are sometime enclosed in a food packaging container. On the other hand, however, in particular, in food products which, after preparation, processing, cooking and the like, arrive on the market based on the premise that they are consumed by consumers in a short period, for example, raw animal meat, fish meat, read-to-eat daily dishes, fresh fish and shellfish, fresh vegetables, fruits, dried fruits, Japanese-style confections and western confectioneries, there has been no simple method for controlling the freshness of the product.

Further, also in products other than food products, for example, in the field of printing ink, the field of electric appliances and mamchinery and appliances, and the field of pharmaceutical preparations and medical supplies, there has been no simple method, for example, for determining quality preservation and for controlling quality, which is effective for simple quality control and is particularly effective for quality control for a short period of time of a few days to a few months.

For example, the above-described Japanese Patent Laid-Open No. 190729/1999 discloses a tape-like stick-on oxygen detecting agent. The tape-like stick-on oxygen detecting agent includes a tape-like oxygen detecting agent (1). The tape-like oxygen detecting agent (1) has been formed by attaching an oxygen detecting composition by coating, printing or impregnation to a non-gas permeable and non-moisture permeable tape-like base material. The tape-like stick-on oxygen detecting agent further includes a plastic single-side adhesive tape (2) having, on its one side, at least a partly transparent adhesive layer with a wider width than the agent (1). The whole oxygen detecting composition attaching surface of the tape-like oxygen defecting agent (1) is adhered via the single-side adhesive tape (2) substantially without air gap. The claimed advantage of this oxygen detecting agent is that the oxygen detecting agent rapidly causes a change in color.upon a change in the concentration of oxygen within the packaging container and a reduction in the oxygen concentration can be easily visually detected without time delay and, thus, the oxygen detecting agent can be utilized for the quality control of deoxidized and packaged food products and the like.

This publication, however, never suggests that, when the number of kinds of products such as food of which the quality is to be controlled and there are various different quality control conditions such as storage temperature and packaging form (for example, vacuum or gas replacement hermetic packaging or air permeable pack packaging) and quality preservation period, how the quality of food or the like can be controlled satisfactorily and easily in relationship with the kind of the food and the quality control conditions for the product.

An object of the present invention is to solve the above problems of the prior art and to provide a method for controlling the quality of a product typified by the control of the freshness of food, in which, when a detecting agent is applied directly or indirectly to a product such as food, for example, consumers or food transactors can very easily determine the freshness of the food as a measure of choice and discrimination of food products or the like, by taking advantage of such a phenomenon that, for example, after display of food on a display shelf in a supermarket or the like, when a predetermined quality preservation period corresponding to the kind of the food for quality control conditions for the food, has elapsed, the detecting agent undergoes a change in color.

Another object of the present invention is to provide a novel oxygen detecting agent, in which, when the oxygen detecting agent is applied directly or indirectly to a product (merchandise) such as food, for example, consumers or food transactors can very easily determine the freshness of food as a measure of choice and discrimination of merchandise or the like, by taking advantage of such a phenomenon that, after display of a product, for example, food, on a display shelf in a supermarket or the like, when a predetermined quality preservation period corresponding to the kind of the food has elapsed, a detecting agent undergoes a change in color, and that is suitable for use in a method for controlling the quality of a product typified by the above control of the freshness of food.

### DISCLOSURE OF THE INVENTION

The method for controlling the quality of a product according to the present invention is characterized in that a product and an oxygen detecting agent having a predetermined sensitivity corresponding to the kind of the product and quality control conditions for the product are provided in combination and the quality of the product is controlled by taking advantage of such a phenomenon that, when a predetermined (quality reservation) period of time corresponding to the kind of the product and the quality control conditions for the product has elapsed, the oxygen detecting agent undergoes a change in color as a result of the detection of the concentration of oxygen, which has infiltrated the oxygen detecting agent, by the oxygen detecting agent.

In the present invention, the combination of the product with the oxygen detecting agent having a predetermined sensitivity corresponding to the kind of the product and the quality control conditions for the product may be contained within one gas-replaced hermetically sealable packaging bag not containing any deoxidant. In this embodiment, the quality of the product can easily be controlled as follows. Specifically, in such a state that a product and an oxygen detecting agent having a predetermined sensitivity corresponding to the kind of the product and quality control conditions for the product are placed in a packaging bag and any deoxidant is not contained within the packaging bag, the air within the packaging bag is replaced by gas. Thereafter, the packaging bag is kept in a hermetically sealed state. In this case, the concentration of oxygen which infiltrates the interior of the packaging bag from the exterior environment with the elapse of a predetermined period of time corresponding to the kind of the product and quality control conditions for the product is detected by the oxygen detecting agent, and, consequently, the oxygen detecting agent undergoes a change in color.

In the present invention, preferably, the product is food and the quality of the product to be controlled is freshness of the food.

In the present invention, preferably, the oxygen detecting agent comprises an oxygen detecting agent body and a film that hermetically covers said body and has a predetermined oxygen permeability and/or carbon dioxide permeability corresponding to the kind of a product such as food and the quality control conditions for the product, such as temperature and ambient gas.

Further, preferably, the oxygen detecting agent is provided in combination with a color sample constructed integrally with or separately from the oxygen detecting agent and indicates at least a color indicative of that the oxygen detecting agent is in an initial state before oxidation, a color indicative of that the product is in the end of use-by date or in such a state that frozen storage should be commenced, and optionally a color indicative of that the product is in a state before use-by date, together with the respective corresponding notations.

In the oxygen detecting agent according to the present invention, preferably, the oxygen detecting agent body has been prepared by impregnating a porous inorganic oxide with an oxygen detecting composition solution and optionally drying the impregnated porous inorganic oxide.

In the present invention, preferably, the gas replacement is carried out by repelling air within the packaging bag and introducing inert gas into the packaging bag.

In the present invention, preferably, the oxygen detecting agent detects oxygen and indicates the detection by taking advantage of a change in color of a redox dye.

In the present invention, preferably, the food is one or at least two articles of food selected from ready-to-eat daily dishes, Japanese unbaked sweets, and western-style confectionary.

Further, in the present invention, preferably, the food is one or at least two articles of food selected from animal meat and fish meat.

In the method for controlling the quality of a product according to the present invention, after display of a product, for example, food, on a display shelf in a supermarket or the like, the detecting agent undergoes a change in color, for example, with the elapse of a predetermined quality preservation period determined depending upon the kind of the product. Therefore, for example, a consumer or a person responsible for handling food can very easily determine the freshness of the food based on a change in color of the detecting agent. Thus, a method for controlling the quality of a product typified by a method for controlling the freshness of food can be provided which can be a measure of merchandise selection and discrimination or the like.

Further, according to the present invention, there is provided an oxygen detecting agent that is suitable for use in the method for controlling the quality of a product, for example, the above method for controlling the freshness of food, and can exhibit the above function.

### BEST MODE FOR CARRYING OUT THE INVENTION

The method for controlling the quality of a product according to the present invention and an oxygen detecting agent used therefor will be specifically described by taking the method for controlling the freshness of food and an oxygen detecting agent used therefor, which are particularly preferred embodiments of the present invention, as an example.

In the method for controlling the freshness of food according to the present invention (first invention), food and an oxygen detecting agent having a predetermined sensitivity corresponding to the kind of the food, for example, whether the food is (i) ready-to-eat daily dishes, Japanese unbaked sweets; western-style confectionary or the like, or (ii) raw animal meat, fish meat or the like, and quality control conditions for the food, for example, the temperature of the food during storage and control of the food (storage temperature), the form of food packaging (for example, vacuum or gas replacement hermetic packaging or air permeable pack packaging), and quality preservation period of the food, are placed in combination, and the oxygen detecting agent detects the concentration of oxygen which enters (infiltrates) the oxygen detecting agent with elapse of a predetermined period determined depending upon the kind of the food and quality control conditions for the product, for example, the elapse of the quality preservation period, and consequently undergoes a change of color, whereby the control of freshness of the food is carried out.

In the second method for controlling the freshness of food according to the present invention (second invention), the combination of the food with the oxygen detecting agent having a predetermined sensitivity corresponding to the kind of the food and the quality control conditions for the food, for example, freshness, may be placed within one gas-replaced packaging bag not containing any deoxidant. In this embodiment, the control of freshness of the food can be carried out as follows. Specifically, in such a state that food and an oxygen detecting agent having a predetermined sensitivity corresponding to the kind of the food and freshness control conditions for the food are placed in a packaging bag and any deoxidant is not contained within the packaging bag, the air within the packaging bag is replaced by gas. Thereafter, the packaging bag is kept in a hermetically sealed state. In this case, the concentration of oxygen or carbon dioxide gas, particularly the concentration of oxygen, which infiltrates the interior of the food packaging bag from the exterior environment with the elapse of a predetermined period of time corresponding to, for example, the kind of the food and freshness control conditions for the food is detected by the oxygen detecting agent, and, consequently, the oxygen detecting agent undergoes a change in color, whereby the control of freshness of the food can be carried out.

In the first and second inventions, after the food of which the freshness is to be controlled is placed under conditions, which cause a deterioration in quality of the food in a relatively short period of time, such as a food display shelf (cabinet) in a supermarket, the oxygen detecting agent undergoes a change in color, for example, due to the elapse of a predetermined quality preservation period, for example, for indicating the expiration of the quality-preservation period. Therefore, for example, a consumer/transactor of the product can easily grasp the quality, and, at the same time, unlike reading of numerical value or character, there is no fear of misreading. Therefore, the false recognition of quality hardly occurs.

### <Oxygen detecting agent>

Preferably, the oxygen detecting agent used in both the method for controlling the freshness of food in the first invention and the method for controlling the freshness of food in the second invention comprises an oxygen detecting agent body, which undergoes a change in color depending upon oxygen permeation (permeability) and/or carbon dioxide permeability, preferably depending upon oxygen permeability, and a film for hermetically covering the body (an oxygen permeability control film), and the film has a predetermined oxygen permeability and/or a carbon dioxide permeability, particularly oxygen permeability, depending upon the kind of a product such as food, of which the quality is to be controlled, and quality control conditions, for example, storage temperature and humidity, kind of replacement gas and gas replacement rate, and the desired freshness of the food.

When the oxygen detecting agent is, for example, in a sheet or tape form, an adhesive layer or a double face adhesive tape may be applied to any one side of the oxygen detecting agent so that the oxygen detecting agent can easily be adhered and fixed onto a desired counter face such as an inner face or an outer face (surface) of a packaging bag. Further, when the oxygen detecting agent is in a tablet form, the tablets may be placed in a transparent air permeable plastic bag or the like.

Regarding the oxygen detecting agent body and the film (oxygen permeability control film), the conventional ones may be used in proper combination by taking into consideration the kind of the product and quality control conditions.

For example, the oxygen detecting agent body may be such that the concentration of oxygen or the presence or absence of oxygen within or in the outside of a packaging vessel for food or the like can be detected by detecting the concentration of oxygen or the presence or absence of oxygen, which is passed or permeated through the oxygen permeability control film, and, in particular, in gas replacement in gas replacement packaging, deoxidation packaging or the like, for example, the fact that oxygen has been satisfactorily removed or that the oxygen concentration is the limitation value can be detected, and, for example, the concentration of oxygen or the presence or absence-of oxygen can be indicated by taking advantage of a color change, that is, a change in hue, tint or lightness.

From the viewpoints of preventing erroneous use of the oxygen detecting agent and properly controlling the quality, preferably, the oxygen detecting agent is placed in combination with a color sample constructed integrally with or separately from the oxygen detecting agent and indicates at least a color indicative of that the oxygen detecting agent is in an initial state before oxidation, a color indicative of that the product is in the end of use-by date or in such a state that frozen storage should be commenced, and optionally a color indicative of that the product is in a state before use-by date, together with notations which explain the state of each food corresponding to a color change of the oxygen detecting agent (for example, blue of methylene blue) and no color change (for example, pink of methylene blue), and the kind of food, of which the quality is to be controlled by the oxygen detecting agent, or quality control conditions (for example, storage temperature of the food, storage time, or packaging conditions, for example, for vacuum hermetic sealing and gas replacement packaging).

Examples of the oxygen detecting agent body include tablet-like molded products, those prepared by impregnating paper, thread, absorbent cotton, porous resin, porous inorganic material, or porous inorganic oxide or other porous member with an oxygen detecting composition solution and optionally subjecting the impregnation product to drying or the like, those prepared by coating an oxygen detecting composition onto the surface of paper or a film and optionally drying the coated paper or film, or those prepared by printing the above composition.

In particular, in applications where lightfastness is required, for example, an oxygen detecting agent for quality control of meat or the like which is displayed on a display shelf which is bright for a few days, the use of a porous member such as a porous inorganic oxide which will be described later is preferred from the viewpoint of maintaining the color tone in a better state after the color change of the oxygen detecting agent.

The oxygen detecting composition may be a conventional one. The oxygen detecting composition, however, is preferably the so-called redox oxygen detecting agent. The redox oxygen.detecting agent is typified by a combination of a specific coloring matter such as methylene blue with a reducing agent for reducing the coloring matter, such as glucose (see, for example, Japanese Patent Laid Open Nos. 39429/2000, 120493/1978, and 60349/1981). This oxygen detecting agent utilizes such a phenomenon that, when oxygen is present, methylene blue per se is oxidized to develop blue while, when oxygen is absent, the coloring matter which has developed blue is reduced with a reducing agent such as glucose (oxygen, which has oxidized the coloring matter, is deprived by glucose which per se is oxidized) and consequently becomes colorless. In general, a coloring matter (for example, red coloring matter such as food red) is incorporated so that, when methylene blue has become colorless (or a light pink color), this can be very clearly learned.

Porous inorganic materials among the porous members include activated carbon, charcoal, and bone black. Porous inorganic oxides include oxides of silicon (Si), aluminum (Al), magnesium (Mg), calcium (Ca), zinc (Zn), barium (Ba), potassium (K) and the like, mixtures of these oxides, and multiple oxides containing these elements.

More specific examples of porous inorganic oxides include silica gel, silica, zeolite, mordenite, bentonite, montmorillonite, (activated) alumina, magnesia, titania, activated clay, clay, slag, bauxite, and porous glass beads.

Among these porous members, porous inorganic oxides are preferred. Further, silica gel, alumina, zeolite, and mordenite are preferred; for example, from the viewpoints of lightfastness, visibility of color tone change, and safety of the oxygen detecting agent.

These porous members may be used solely or in a combination of two or more of them.

Oxygen permeability control films (films) include, for example, transparent films of polyethylene, polypropylene, polyester, polyamide, polycarbonate, cellulose acetate, and cellophane. So far as these films are transparent, they may have been stretched, or a metal or the like may have been provided, for example, by vapor deposition on the surface thereof, or these films and the like may have been stacked on top of each other in any combination.

Specifically, for example, conventional transparent films having various oxygen permeabilities (and/or carbon dioxide permeabilities) exemplified by the following (i) to (xi) may be used as the oxygen permeability control film (for example, see "Shokuhin Hoso Binran (Food Packaging Encyclopedia)", p. 495, table 2, published by Japan Packaging Insutitute):
(i) : a laminated film of OPP (OPP: biaxially oriented polypropylene, thickness 20 µm/oriented HDPE (HDPE: high density polyethylene, thickness 45 µm)/LDPE (LDPE: low density polyethylene, thickness 30 µm), having an oxygen transmission rate (oxygen permeability) of 2000 ml/m²·24 hr·atm (temperature 30°C, wet) and a moisture permeability of 1 g/m²·24 hr (40°C, 90% RH) ;
(ii): a laminated film of OPP (OPP: biaxially oriented polypropylene, thickness 20 µm)/CPP (CPP: non-oriented polypropylene film, thickness 30 µm), having an oxygen transmission rate of 2000 ml/m²·24 hr·atm (temperature 30°C, wet) and a moisture permeability of 6 g/m²·24 hr (40°C, 90% RH) ;
(iii): a laminated film of cellophane (thickness 20 µm)/LDPE (LDPE: low density polyethylene, thickness 40 µm), having an oxygen transmission rate of 1000 ml/m²·24 hr·atm (temperature 30°C, wet) and a moisture permeability of 15 g/m²·24 hr (40°C, 90% RH) ;
(iv): a laminated film of PVDC (PVDC: polyvinylidene chloride, thickness 3 µm) coated ONylon (ONylon: oriented nylon: thickness 18 µm)/EVA (EVA: ethylene/vinyl acetate copolymer resin, thickness 5D µm), having an oxygen transmission rate of 12 ml/m²·24 hr·atm (temperature 30°C, wet) and a moisture permeability of 13 g/m²·24 hr (40°C, 90% RH) ;
(v): a laminated film of PVDC (PVDC: polyvinylidene chloride, thickness 3 µm) coated PET (thickness 15 µm) /CPP (non-oriented polypropylene film, thickness 50 µm), having an oxygen transmission rate of 12 ml/m²·24 hr-atm (temperature 30°C, wet) and a moisture permeability of 8 g/m²·24 hr (40°C, 90% RH) ;
(vi): a laminated film of PVDC (polyvinylidene chloride, thickness 3 µm) coated cellophane (thickness 23 µm)/LDPE (low density polyethylene), having an oxygen transmission rate of 12 ml/m²·24 hr·atm (temperature 30°C, wet) and a moisture permeability of 10 g/m²·24 hr (40°C, 90% RH) ;
(vii): a laminated film of PVDC (polyvinylidene chloride, thickness 3 µm) coated OPP (biaxially oriented polypropylene, thickness 23 µm)/LDPE (low density polyethylene, thickness 40 µm), having an oxygen transmission rate of 12 ml/m²·24 hr-atm (temperature 30°C, wet) and a moisture permeability of 5 g/m²·24 hr (40°C, 90% RH) ;
(viii): a laminated film of PET (thickness 12 µm)/aluminum deposited LDPE (low density polyethylene, thickness 30 µm), having an oxygen transmission rate of 10 ml/m²·24 hr·atm (temperature 30°C, wet) and a moisture permeability of 1 g/m²·24 hr (40°C, 90% RH) ;
(ix): a laminated film of aluminum deposited PET (thickness 12 µm)/LDPE (low density polyethylene, thickness 40 µm), having an oxygen transmission rate of 2 ml/m²·24 hr·atm (temperature 30°C, wet) and a moisture permeability of 1 g/m²·24 hr (40°C, 90% RH);
(x) : a laminated film of PVDC (thickness 3 µm) coated oriented PVA (thickness 15 µm)/LDFB (low density polyethylene, thickness 70 µm), having an oxygen transmission rate of 2 ml/m²·24 hr·atm (temperature 30°C, wet) and a moisture permeability of 5 g/m²·24 hr (40°C, 90% RH); and
(xi) : a laminated film of PVDC (thickness 12 µm) coated cellophane (thickness 32 µm) /ZDPE (low density polyethylene, thickness 40 µm), having an oxygen transmission rate of 1.5 ml/m²·24 hr·atm (temperature 30°C, wet) and a moisture permeability of 2.5 g/m²·24 hr (40°C, 90% RH).

Among the films which may be in a laminated form, the use of transparent films having a low oxygen permeability (oxygen transmission rate) of 1.0 to 100 ml/m²·24 hr·atm (temperature 30°C, wet), preferably 1.0 to 40 ml/m²·24 hr·atm (temperature 30°C, wet), particularly preferably 1.0 to 15 ml/m²·24 hr·atm (temperature 30°C, wet) exemplified by the above (iv) to (xi) is effective, for example, in controlling the freshness of food in a low sensitivity state by applying an oxygen detecting agent to an outer face (pack outer face) in direct contact with the atmospheric air in a raw animal meat or fish meat pack which will be described later, or a small bag inner face for a raw meat pack (pack inner face) through which oxygen or the atmospheric air can substantially freely go in and out.

Further, among these films, the use of transparent films particularly having a high oxygen permeability of 500 to 3000 ml/m²·24 hr·atm (temperature 30°C, wet), preferably 800 to 2500 ml/m²·24 hr·atm (temperature 30°C, wet), particularly preferably 1500 to 2300 ml/m²·24 hr·atm (temperature 30°C, wet) exemplified by the above (i) to (iii) is effective in controlling the freshness of food or the like in a high sensitivity state by applying an oxygen detecting agent to a hermetically sealable pack on its inner face (the infiltration of oxygen therethrough being suppressed or prevented) in a ready-to-eat daily dish or a gas replaced lunch box (pack) containing a ready-to-eat daily dish which will be described later.

The kind of the film and the like may be properly selected, for example, depending upon the kind of the food and packaging form.

### (Control of freshness of food such as ready-to-eat daily dish or lunch box (pack) containing ready-to-eat daily dish)

The present invention will be described in more detail with reference to the following embodiments. As described above, for example, for food such as ready-to-eat daily dishes or lunch boxes (packs) containing a ready-to-eat daily dish, after the preparation thereof, they are displayed on food display shelves in supermarkets or convenience stores or the like at room temperature (15 to 25°C) to "room temperature - 5°C" for a short period of time of about 2 or 3 days and are in many cases purchased and consumed by consumers in a few days.

For the ready-to-eat daily dishes or lunch boxes (packs) containing a ready-to-eat daily dish, an example of a process from preparation to consumption is as follows. For example, a ready-to-eat daily dish, rice and the like are packed, for example, within a kitchen in food manufacturing facilities at room temperature under the atmospheric pressure. An oxygen detecting agent is enclosed in the pack. The air within the pack is replaced, for example, by inert gas, for example, a mixed gas composed of nitrogen gas (Nz) and carbon dioxide (CO₂) (for example, N₂/CO₂ (mixing volume ratio) = 1/0.8 to 1/1.2). In such a gas-replaced and hermetically sealed state that the concentration of oxygen gas immediately after gas replacement is generally not more than 0.2% and, in many cases, about 0.2 to 0.3%, the packs are distributed in a distribution process.

The inert gas is not limited to the above examples, and conventional inert gases such as nitrogen gas, carbon dioxide, argon gas, and helium gas may also be used. These inert gases may also be used in a combination of two or more of them.

According to the construction of the present invention, after display of the food on a display shelf or the like in a supermarket, on the second to third day from the preparation of the packed food, that is, after the elapse of a predetermined quality preservation period corresponding to the kind of the food where the concentration of oxygen within the packs reaches, for example, about 1%, the oxygen detecting agent enclosed in the packs (or applied to the inner wall of the packs) undergoes a color change.

Thus, for example, consumers and food transactors can very easily determine the freshness of the food and can utilize the above phenomenon as a measure of merchandise choice and discrimination or the like.

For example, when the oxygen detecting agent body as one member constituting the oxygen detecting agent is a red coloring matter-containing substrate impregnated with methylene blue, as soon as the ambient concentration of oxygen increases and reaches about 0,9 to 1.2% (for example, 1.0%), the color changes from red to blue.

Accordingly, when the object food is, for example, the above ready-to-eat daily dish or lunch box containing a ready-to-eat daily dish, a film having an oxygen transmission rate (oxygen permeability) of 1000 to 3000 ml/m²·24 hr·atm, preferably 1500 to 2500 ml/m²·24 hr·atm, may be adopted as the film (oxygen permeability control film) for hermetically covering the oxygen detecting agent body.

Preferably, the oxygen permeability control film is resistant to (insoluble in) water (cooking liquid), edible oils, alcohols and the like contained in the food in the pack, and examples thereof include polypropylene (OP)/polyethylene (PE) laminated films and polyester (PET)/polyethylene (PE) laminated films.

When the oxygen detecting agent body is hermetically covered with the oxygen permeability control film having the above oxygen permeability, as soon as the above number of days (for example, two to three days after the preparation of the food) elapses under the above room temperature (for example, a temperature around 15 to 25°C), for example, temperature within a ready-to-eat daily dish display case in a supermarket, the oxygen detecting agent body can undergo a change in color from red to blue. Therefore, the freshness of the ready-to-eat daily dish or the lunch box containing a ready-to-eat daily dish (quality preservation period) can be easily and reliably determined.

Other food of which the freshness can be controlled, particularly under temperature conditions close to room temperature in a short period, as in the ready-to-eat daily dish or lunch box containing a ready-to-eat daily dish includes Japanese-style confectionary and western-style confectionary. Dried fruits, cereals and the like may also be objects of the freshness control by setting proper conditions.

### (Control of freshness of food such as raw animal meat)

When the object food is, for example, raw animal meat, the following embodiment is often adopted. Specifically, an oxygen detecting agent is enclosed in combination with divided raw animal meat packs in a hermetically sealable exterior packaging bag, and, if necessary, a deoxidizer is also enclosed within the exterior packaging bag. The air within the exterior packaging bag is replaced, for example, by carbon dioxide (CO₂) gas so that the concentration of oxygen gas immediately after the gas replacement is generally not more than 0.05% and in most cases about 0.001 to 0.05%. In some cases, transport/storage is then carried out for several tens of days (for example, 40 days) at about 0°C (embodiment A).

Further, for the raw animal meat, the following embodiment may also be adopted. Specifically, an oxygen detecting agent is enclosed in combination with divided raw animal meat packs in a hermetically sealable exterior packaging bag, and, if necessary, a deoxidizer is also enclosed within the exterior packaging bag. The inside of the exterior packaging bag is deaerated, and, in some cases, for example, storage in a frozen state for a long period of time at such a temperature that does not substantially cause a deterioration in quality (for example, about -40°C to -18°C), or chilled transport at that temperature is carried out (embodiment B).

After transport and storage under such various conditions (embodiment A or embodiment B), the hermetically sealed exterior packaging bag is opened and removed, and the raw animal meat is displayed on a food display shelf (cabinet) in a supermarket or the like. In this case, the oxygen detecting agent which has been placed in combination with the divided pack comes into direct contact with the atmospheric air and begins to absorb ambient oxygen (oxygen in the air). After the elapse of the above predetermined number of days (for example, about 3 to 4 days at a temperature around 2 to 10°C), the oxygen detecting agent body can detect that oxygen has been passed through and infiltrated the oxygen permeability control film and the concentration of oxygen which has entered the interior (on the oxygen detecting agent body side) has reached a predetermined value. The detection of the oxygen concentration can be learned from a color change of the oxygen detecting agent from red to blue. Thus, the freshness of the raw meat, that is, whether or not the quality preservation period has expired, can be easily and reliably determined.

In this case, a film having an oxygen transmission rate (oxygen permeability) of 1 to 50 ml/m²·24 hr·atm, preferably 5 to 20 ml/m²·24 hr·atm, may be adopted as the film for hermetically covering the oxygen detecting agent body (oxygen permeability control film).

Such oxygen permeability control films include polyethylene terephthalate (PET)/polyacrylonitrile (PAN) laminated film, and vinyl chloride coated polyethylene film (KNY/PE).

When the oxygen detecting agent body is hermetically covered with a film having the above oxygen transmission rate, even in the case where, after packaging of the raw animal meat, the oxygen detecting agent is already applied to the outer surface of divided packs before transport and storage, or is enclosed in air permeable divided packs, since the oxygen detecting agent is placed together with the raw animal meat in the divided pack and, during transport and storage, is further placed within an exterior packaging bag for transport and storage in the low oxygen content state, the quality of the raw meat in the divided pack is not substantially deteriorated. Therefore, during this period, the oxygen detecting agent does not cause a color change, indicating that the raw meat is fresh.

In the present invention, however, when the exterior packaging bag for maintaining the low oxygen content state is once opened and the oxygen detecting agent is exposed to the atmospheric air, as the above number of days (3 to 4 days), for example, at 2 to 10°C (for example, 6°C) elapse, the oxygen detecting agent absorbs a predetermined amount of oxygen contained in the atmospheric air, resulting in a color change of the oxygen detecting agent body from red to blue.

The color change conditions of the oxygen detecting agent can be varied by varying the oxygen permeability and/or carbon dioxide gas permeability, particularly oxygen permeability, of the film covering the oxygen detecting agent body (oxygen permeability control film) to a predetermined value depending upon the kind of a product of which the quality is to be controlled, such as food, and quality control conditions such as temperature, humidity, the kind of replacement gas and gas replacement rate, and freshness. This can realize the determination of the freshness of raw animal meat (quality preservation period) in an easy and reliable manner.

Other articles of food of which the freshness can be controlled in the same manner as the above raw animal meat include fishery products such as lobsters or prawns or shrimps, crab, oyster, and fish (which are often collectively referred to as "fish meat").

In the above description, preferred embodiments of the present invention have been described by taking, as examples, the control of freshness of the ready-to-eat daily dish or the lunch box containing a ready-to-eat daily dish using, as a film for hermetically covering the oxygen detecting agent body (oxygen permeability control film), a film having an oxygen transmission rate (oxygen permeability) of 1000 to 3000 ml/m²·24 hr·atm, preferably 1500 to 2500 ml/m²·24 hr·atm, or the control of freshness of raw animal meat using, as an oxygen permeability control film, a film having an oxygen transmission rate (oxygen permeability) of 1 to 50 ml/m²·24 hr·atm, preferably 5 to 20 ml/m²·24 hr·atm. The present invention, however, is not limited to these embodiments only. For example, the control of quality of various objects can be carried out under various conditions by hermetically covering the oxygen detecting agent body with conventional films having various oxygen permeabilities and/or carbon dioxide gas permeabilities (see, for example, "Shokuhin Hoso Binran (Food Packaging Encyclopedia)", p. 495, Table 2).

As described above in detail, the method for controlling the quality of a product according to the present invention typified by the method for controlling the freshness of food can provide a method for controlling the quality of a product typified by a method for controlling the freshness of a product (food) in which, for example, consumers or product transactors (for example, food transactors) can very easily determine quality such as freshness of the product (food) as a measure of merchandise choice and discrimination or the like, by taking advantage of such a phenomenon that, after display of a product, for example, food, on a display shelf in a supermarket or the like, when a predetermined quality preservation period corresponding to the kind of the product (for example, food) and the quality control conditions for the product has elapsed, an oxygen detecting agent undergoes a change in color.

In the present invention, products as objects of which the quality can be controlled are not limited to the above food only, and other products to which the present invention can be applied include: articles of food which undergo a deterioration in taste, odor, or flavor due to oxidation degradation or the like, for example, rehydratable noodle, dried bread-like pieces of wheat gluten, spices, or sweet stuff; articles of taste which undergo a deterioration in taste due to oxidation degradation, for example, cigarettes; pharmaceutical preparations, particularly pharmaceutical preparations in which the quality and efficacy of medicinal ingredients greatly depending upon the storage period, temperature and the like, for example, viable cells (for example, lactic acid bacteria and vaccine), blood products, crude drug-containing pharmaceutical preparations; milky lotions and other cosmetic preparations containing natural product-derived ingredients and the like in which the odor, color, skin beautiful effect and the like are likely to be lost by oxidation degradation; articles of which the quality and efficacy greatly depend upon storage period, temperature, oxidation degradation and the like, for example, contact lenses and cleaning liquids and preserving liquids for contact lenses; and components and merchandises in which rust prevention and the like are required from the viewpoints of function retention, appearance and the like, for example, clocks and watches and electronic components, precision machines and instruments, accessories, and artistic handicrafts.

The method for controlling the quality of a product according to the present invention can provide a method for controlling the quality of a product typified by a method for controlling the freshness of food in which, for example, consumers or food transactors can very easily determine the freshness of food as a measure of merchandise choice and discrimination or the.like, by taking advantage of such a phenomenon that, after display of a product, for example, food, on a display shelf in a supermarket or the like, when a predetermined quality preservation period corresponding to the kind of the food has elapsed, a detecting agent undergoes a change in color.

In particular, immediately before the display of food, an oxygen detecting agent having a specific oxygen permeability selected depending upon the kind of the food and storage control conditions is applied, for example, to the inner face of a packaging bag which has been subjected to gas replacement or the like for a reduction in oxygen gas content (packaging bag on its inner face which is isolated from the atmospheric air), for example, consumers or persons responsible for controlling food in a supermarket or the like can simply grasp the freshness and the like of the food and can reduce a fear of false recognition of the quality, without the need to check a label attached to the food packaging bag, indicating the date of packing, quality preservation period and the like.

Further, the present invention provides an oxygen detecting agent that is suitable for use in a method for controlling the quality of a product, such as the above method for controlling the freshness of food and can exhibit the above function.

### EXAMPLES

The following Examples further illustrate the present invention. However, it should be noted that the present invention is not limited to these Examples only.

### <Oxygen detecting agent for control of freshness of foods such as ready-to-eat daily dish and lunch box containing ready-to-eat daily dish (a)>

(i) Oxygen detecting agent body: one prepared by impregnating a substrate (a carrier) with methylene blue or the like as a redox dye and supporting the methylene blue or the like on the substrate.
(ii) Oxygen permeability control film (film): a laminated transparent film of polypropylene (OP)/polyethylene (PE) having an oxygen permeability (oxygen transmission rate) of 2000 ml/m²·24 hr-atm.
(iii) Preparation of oxygen detecting agent: An aqueous solution containing glucose as a reducing agent, potassium hydroxide as an alkali metal compound, and methylene blue as a redox dye to be reduced by the reducing agent, and optionally food red as nonreducing coloring matter is supported on a hygroscopic thin sheet carrier, and the sheet is then dried and is sealed in the above transparent film (oxygen permeability control film).
(iv) Dimension of oxygen detecting agent: 43 mm in length × 30 mm in width × 0.5 mm in thickness.

### <Oxygen detecting agent for control of freshness of foods such as ready-to-eat daily dish and lunch box containing ready-to-eat daily dish (a-1)>

An oxygen detecting agent was prepared in the same manner as in oxygen detecting agent (a), except that, in the step of preparation (iii) of the oxygen detecting agent in the above <Oxygen detecting agent for control of freshness of foods such as ready-to-eat daily dish and lunch box containing ready-to-eat daily dish (a)>, silica gel was used instead of the "hygroscopic thin sheet carrier":
(i) Oxygen detecting agent body: the same as in oxygen detecting agent (a) (i), except for the substrate.
(ii) Oxygen permeability control film (film): the same as in oxygen detecting agent (a) (ii).
(iii) Preparation of oxygen detecting agent: An aqueous solution containing glucose as a reducing agent, potassium hydroxide as an alkali metal compound, and methylene blue as a redox dye to be reduced by the reducing agent, and optionally food red as nonreducing coloring matter is supported on silica gel, followed by drying. Thereafter, the resultant granules/powder are sealed in the above transparent film (oxygen permeability control film).
(iv) Dimension of oxygen detecting agent: the same as in oxygen detecting agent (a) (iv).

### <Oxygen detecting agent for control of freshness of raw animal meat (b)>

(i) Oxygen detecting agent body: one prepared by impregnating a substrate with methylene blue or the like as a redox dye.
(ii) Oxygen permeability control film (film) : a laminated film of polyethylene terephthalate (PET)/polyacrylonitrile (PAN) having an oxygen permeability (oxygen transmission rate) of 10 ml/m²·24 hr·atm.
(iii) Preparation of oxygen detecting agent: the same as in oxygen detecting agent (a) (iii).
(iv) Dimension of oxygen detecting agent: 43 mm in length x 30 mm in width x 0.5 mm in thickness.

### <Oxygen detecting agent for control of freshness of raw animal meat (b-1)>

An oxygen detecting agent was prepared in the same manner as in oxygen detecting agent (b), except that, in the step of preparation (iii) of the oxygen detecting agent in the above <Oxygen detecting agent for control of freshness of raw animal meat (b)>, silica gel was used instead of the "hygroscopic thin sheet carrier":
(i) Oxygen detecting agent body: the same as in oxygen detecting agent (b) (i), except for the substrate.
(ii) Oxygen permeability control film (film): the same as in oxygen detecting agent (b) (ii).
(iii) Preparation of oxygen detecting agent: An aqueous solution containing glucose as a reducing agent, potassium hydroxide as an alkali metal compound, and methylene blue as a redox dye to be reduced by the reducing agent, and optionally food red as nonreducing coloring matter is supported on silica gel, followed by drying. Thereafter, the resultant granules/powder are sealed in the above transparent film (oxygen permeability control film).
(iv) Dimension of oxygen detecting agent: the same as in oxygen detecting agent (b) (iv).

### [Example 1]

### (Control of freshness of foods such as ready-to-eat daily dish and lunch box containing ready-to-eat daily dish)

A lunch box (polystyrene pack, thickness 100 µm, a commercially available product) containing ready-to-eat daily dish was prepared at room temperature (20°C). Thereafter, oxygen detecting agent (a) {prepared by hermetically sealing the oxygen detecting agent body in an OP/PE laminated transparent film having an oxygen permeability of 2000 ml/m²·24 hr·atml was placed in the pack. The air within the pack was replaced by a mixed gas composed of nitrogen gas (N₂) and carbon dioxide (CO₂) (for example: N₂/CO₂ (mixing volume ratio) = 1.0/1.0), and the pack was hermetically sealed in a film (oxygen permeability: 10 ml/m²·24 hr·atm, thickness 80 µm, nylon (R)./polyethylene laminated film, a commercially available product).

Two hr after the gas replacement within the pack, the concentration of oxygen gas within the pack was measured with an oximeter (manufactured by Toray Engineering Co., Ltd.). As a result, it was found that the oxygen gas concentration was 0.2% and the color of the oxygen detecting agent was pink.

Sazbsequently, the above food was stored on a shelf having a brightness of 1000 luxes at room temperature (20°C) in the same manner as in a display shelf provided in a supermarket.

On the second day after the preparation of the lunch box containing ready-to-eat daily dish, that is, after the expiration of the quality preservation period of the lunch box containing ready-to-eat daily dish (two days after the preparation), the concentration of oxygen within the pack reached 1.0%, and it was found that the color of the oxygen detecting agent applied to the upper surface within the pack (ceiling plane) changed from red to blue.

Based on this change in color, a consumer could very easily determine the freshness of the food. In this stage, any abnormal appearance was not observed in this food, and the food was still in an eatable state.

### [Example 2]

### (Control of freshness of food such as raw animal meat)

Raw beef (10 kg) was divided into 500 g parts that were each packed (polystyrene pack body (thickness 100 µm)) to prepare 20 individual packs which were then simply packaged using a film (a vinylidene chloride wrap film, a commercially available product). Subsequently, oxygen detecting agent (b) {prepared by hermetically covering an oxygen detecting agent body by a PET/PAN laminated transparent film having an oxygen permeability of 10 ml/m²·24 hr·atm} was enclosed in a wrapping packaging bag (oxygen permeability: 10 ml/m²·24 hr·atm, thickness 100 µm, nylon/polyethylene (PE) laminated film, a commercially available product) in such a state that a combination of each divided pack (raw animal meat) with the oxygen detecting agent (applied to the outer surface of the divided pack). If necessary, a deoxidant was also enclosed in the wrapping packing bag. Thereafter, the air within the wrapping packaging bag was replaced by carbon dioxide gas (CO₂). Immediately after the gas replacement, the concentration of oxygen gas within the wrapping packaging bag was measured with an oximeter (manufactured by Toray Engineering Co., Ltd.) and was found to be 0.01%.

Subsequently, the packed raw animal meat was stored in a dark refrigerator at 0°C for 40 days, during which period the color of the oxygen detecting agent remained unchanged and was pink.

Thereafter, the packed raw animal meat was taken out of the refrigerator, and the wrapping packaging bag for the raw animal meat was cut and removed. The raw animal meat, which was divided into individual packs and in which the oxygen detecting agent was applied to the outer surface of the packs (the divided pack in its outer surface in direct contact with indoor air), was maintained for three days at the same temperature as the temperature within a food display shelf (cabinet) in supermarkets or the like, that is, 6°C, under a brightness of 1000 luxes.

Immediately after display on the food display shelf (cabinet) at 6°C, the color of the oxygen detecting agent applied to the outer surface of the individual packed raw animal meat was pink..

On the third day after the initiation of holding of the divided packed raw meat at the temperature within the food display cabinet, that is, 6°C, it was found that the color of the oxygen detecting agent in direct contact with the air changed to blue due to absorption of oxygen surrounding the oxygen detecting agent (in the air).

On that day (the day on which color change of the oxygen detecting agent to blue was observed), the packs of raw animal meat were opened for inspection of odor, color, taste and the like of the meat contained within the packs. As a result, any abnormal phenomenon was not observed.

Thereafter, this oxygen detecting agent. (b) was allowed to remain applied to the outer surface of the packs, and the packs were placed side-by-side on the food display shelf under the same brightness as described above, that is, 1000 luxes. As a result, a Lendency was observed that blue tone color of methylene blue gradually faded and the color changed to a reddish color which a color tone of food red contained in the oxygen detecting agent.

### [Example 3]

### (Control of freshness of food such as raw animal meat)

The procedure of Example 2 was repeated, except that oxygen detecting agent (b-1) was used instead of oxygen detecting agent (b).

As a result, immediately after display on the food display shelf (cabinet) at 6°C, the color of the oxygen detecting agent (b-1) applied to the outer surface of the individual packed raw animal meat was pink.

On the third day after the initiation of holding of the divided packed raw meat at the temperature within the food display cabinet, that is, 6°C, it was found that the color of the oxygen detecting agent in direct contact with the air changed to blue due to absorption of oxygen surrounding the oxygen detecting agent (in the air).

On that day (the day on which color change of the oxygen detecting agent to blue was observed), the packs of raw animal meat were opened for inspection of odor, color, taste and the like of the meat contained within the packs. As a result, any abnormal phenomenon was not observed.

Thereafter, this oxygen detecting agent (b-1) was allowed to remain applied to the outer surface of the packs, and the packs were placed side-by-side on the food display shelf under the same brightness as described above, that is, 1000 luxes. As a result, unlike Example 2 in which oxygen detecting agent (b) was used, even on the fourth or fifth day after the start of the display, the blue tone color of methylene blue substantially remained unfaded.

## Claims

1. A method for controlling the quality of a product, **characterized in that** a product and an oxygen detecting agent having a predetermined sensitivity corresponding to the kind of the product and quality control conditions for the product are provided in combination and the quality of the product is controlled by taking advantage of such a phenomenon that, when a predetermined period of time corresponding to the kind of the product and the quality control conditions for the product has elapsed, the oxygen detecting agent undergoes a change in color as a result of the detection of the concentration of oxygen, which has infiltrated the oxygen detecting agent, by the oxygen detecting agent.

2. The method for controlling the quality of a product according to claim 1, **characterized in that** the combination of the product with the oxygen detecting agent having a predetermined sensitivity corresponding to the kind of the product and the quality control conditions for the product is contained within one gas-replaced hermetically sealable packaging bag not containing any deoxidant.

3. The method for controlling the quality of a product according to claim 1 or 2, **characterized in that** said oxygen detecting agent comprises an oxygen detecting agent body and a film that hermetically covers said body and has a predetermined oxygen permeability and/or carbon dioxide permeability corresponding to the kind of the product and the quality control conditions for the product.

4. The method for controlling the quality of a product according to any of claims I to 3, **characterized in that** said product is food and the quality of the product to be controlled is freshness of the food.

5. The method for controlling the quality of a product according to any of claims 2 to 4, **characterized in that** said gas replacement is carried out by expelling air within the hermetically sealable packaging bag and then introducing inert gas into the bag.

6. The method for controlling the quality of a product according to claim 4, **characterized in that** said food is one or at least two articles of food selected from ready-to-eat daily dishes, Japanese unbaked sweets, and western-style confectionary.

7. The method for controlling the quality of a product according to claim 4, **characterized in that** said food is one or at least two articles of food selected from animal meat and fish meat.

8. An oxygen detecting agent **characterized by** comprising an oxygen detecting agent body and a film that hermetically covers said body and has a predetermined oxygen permeability and/or carbon dioxide permeability corresponding to the kind of a product, of which the quality is to be controlled, and the quality control conditions for the product.

9. The oxygen detecting agent according to claim 8, **characterized in that** said oxygen detecting agent is provided in combination with a color sample constructed integrally with or separately from said oxygen detecting agent and indicates at least a color indicative of that the oxygen detecting agent is in an initial state before oxidation, a color indicative of that the product is in the end of use-by date or in such a state that frozen storage should be commenced, and optionally a color indicative of that the product is in a state before use-by date, together with the respective corresponding notations.

10. The oxygen detecting agent according to claim 8 or 9, **characterized in that** said oxygen detecting agent body has been prepared by impregnating a porous inorganic oxide with an oxygen detecting composition solution and optionally drying the impregnated porous inorganic oxide.
